# DEMANDE DE BREVET EUROPEEN

(11) **EP 4 717 210 A1**
(43) Date de publication de la demande: **01.04.2026**
(21) Numéro de dépôt: 24202390.1
(22) Date de dépôt: 25.09.2024
(51) Int. Cl.: A61B 17/88, A61F 2/46, A61F 2/44

(54) **IMPLANT OSSEUX EXPANSIBLE DE CHIRURGIE ORTHOPÉDIQUE VÉTÉRINAIRE, SYSTÈME ORTHOPÉDIQUE ET PROCÉDÉ DE FABRICATION DE L'IMPLANT**

(71) Demandeur: IN LIFE VET SA, 1630 Bulle (CH)
(72) Inventeur: LACAZE, Guillaume, 1278 La Rippe (CH)
(74) Mandataire: Gsmart

(57) **Abrégé**

La présente invention concerne un Implant osseux expansible de chirurgie orthopédique vétérinaire, Système orthopédique et Procédé de fabrication de l'implant, pour la restauration de volume et/ou géométrie d'un os, par une expansion entre une configuration repliée et une configuration déployée, au moins deux faces de l'implant comportant chacune un plateau (13, 14) pour le contact avec les tissus osseux, chacun des plateaux comportant une portion centrale (130, 140) reliée, par l'intermédiaire d'au moins une charnière, à au moins une paire de bras de support (131, 141), un bras de chaque paire étant relié par une charnière à l'extrémité distale (11) tandis que l'autre bras est relié par une charnière à l'extrémité proximale (12), le rapprochement entre les extrémités distale (12) et proximale (11) engendrant le pivotement des bras de support (131, 141) provoquant l'éloignement des plateaux (13, 14) l'un par rapport à l'autre et, par conséquent, l'expansion de l'implant
caractérisé en ce que :
- au moins deux autres faces de l'implant sont couvertes de manière étanche par au moins une feuille (10) par face, en alliage biocompatible de métaux,
- ladite feuille (10) est déformable plastiquement pour permettre l'expansion de l'implant, la surface totale de ladite feuille étant supérieure ou égale à la surface latérale de l'implant en configuration déployée de façon à former un compartiment étanche apte à accueillir un fluide à l'intérieur de la cavité obtenue par l'expansion de l'implant

## Description

La présente demande se rapporte au domaine de la chirurgie, en particulier la chirurgie orthopédique vétérinaire, et notamment du traitement d'une structure osseuse affaissée, par une restauration du volume (ou redressement) de cette structure osseuse. La présente demande concerne en particulier un implant et son procédé de fabrication, ainsi qu'un système pour la restauration de structure osseuse, notamment dans le rachis pour le traitement (souvent appelé « réduction ») des fractures de compression, notamment vertébrales (VCF pour l'anglais « Vertébral Compression Fracture »).

Dans ce domaine, le problème de la restauration du volume de structure osseuse qui s'est affaissée est bien connu et la littérature abonde de solutions utilisant des implants expansibles capables de passer d'une configuration repliée à une configuration déployée pour restaurer la hauteur de la structure osseuse, de préférence en combinaison avec une injection de ciment de substitution osseuse, dit ciment (ou ciment osseux). De nombreux ciments sont connus et ils présentent tous l'avantage d'être injectables à l'état liquide ou visqueux pendant une certaine durée, puis de durcir (par polymérisation) à l'intérieur de la structure osseuse pour la stabiliser.

Un problème majeur dans ce domaine concerne l'expansion de l'implant pour restaurer une hauteur au tissu osseux lésé. De nombreuses solutions sont connues de l'art antérieur, comme notamment les demandes de brevet EP3086729, US 11540926, EP3747385, EP2572680, EP3958752, EP2693967, EP2405835, US9579130, EP4216836, WO2023122005, WO2022162418, EP3843668 ou US10945861 mais ces solutions présentent divers problèmes de difficulté de manipulation pour le déploiement, de stabilité et de fiabilité une fois déployés. De plus, ces solutions connues s'accompagnent généralement d'une injection de ciment osseux mais ne fournissent aucun enseignement relatif à la fuite de ciment en dehors de l'implant, alors qu'une telle fuite peut être préjudiciable pour les tissus environnants, voire même l'organisme tout entier si les substances chimiques du ciment envahissent le système sanguin. En effet, le ciment comporte en général une ou plusieurs substances chimiques polymérisables, par exemple comme le poly(méthacrylate de méthyle) (PMMA, pour l'anglais poly-methyl-methacrylate) et éventuellement des additifs. De plus, la température atteinte lors de la polymérisation du ciment n'est pas inoffensive car elle est généralement supérieure à 60°.

Il est connu de l'art antérieur, notamment des document EP1308134, US9510877, US8936627 ou EP2467099, des dispositifs de redressement et stabilisation (ou réduction de fracture d'os), notamment du rachis sous la forme de stent, ou sous forme de ballons ou de sacs gonflables poreux comme dans les documents EP1408888 ou EP1379185, éventuellement équipé de plateaux de support comme dans le document US20060100706. De nombreux documents proposent ce genre de stent, c'est-à-dire d'endoprothèse déformable similaire aux endoprothèses, extenseurs ou tuteurs vasculaires, qui sont généralement sous la forme d'un corps tubulaire maillé, le plus souvent métallique et déformable par l'introduction d'un ballon gonflable pour dilater le corps en écartant les mailles, le ballon étant ensuite retiré pour permettre une injection de ciment, qui durcit et forme ainsi une structure de redressement et de stabilisation. Cependant, ces dispositifs présentent l'inconvénient de nécessiter une implantation en deux temps avec le gonflage du ballon puis l'injection de ciment, ce qui ralentit et complexifie l'opération mais présente également un risque d'affaissement du dispositif entre le dégonflage du ballon et le remplissage du stent par le ciment. D'autre part, ces solutions présentent l'inconvénient de ne pas répondre au problème majeur des fuites de ciment.

Il est également connu, notamment des documents EP1938765, EP2351539 ou WO200434924, des solutions utilisant des implants à structure maillée, en métal à mémoire de forme, qui est contrainte dans une forme repliée pour l'insertion dans le tissu osseux et capable de s'expandre spontanément, lors du relâchement de la contrainte et/ou sous l'effet de la chaleur. Ces solutions présentent l'inconvénient d'une utilisation d'alliages coûteux et une fabrication complexe pour obtenir une mémoire de forme adéquate qui convienne à l'implantation visée, ce qui implique un surcoût en multipliant le nombre d'implants différents nécessaires pour couvrir les différents cas pathologiques, notamment par l'amplitude de la déformation dont est capable le matériau à mémoire de forme. De plus, la force exercée par le retour du métal à sa forme non contrainte n'est souvent pas suffisante pour redresser correctement la structure osseuse qui s'est affaissée, ou du moins limitative. D'autre part, ces solutions présentent également l'inconvénient de ne pas répondre au problème majeur des fuites de ciment.

Il est également connu de l'art antérieur, notamment des documents EP2405835, US9579130, EP2572680 ou EP1956990 des solutions utilisant des implants expansibles utilisant un mécanisme de levier, à la manière d'un cric de voiture (« jack » en anglais) pour restaurer la structure osseuse à une hauteur déterminée. Ces solutions présentent l'avantage de ne pas risquer d'affaissement contrairement à un sent déployés par un ballon qui est retiré avant l'injection de ciment, mais ils présentent également l'inconvénient d'une implantation en deux temps et que les dimensions de la surface de support pour exercer la force d'expansion sur le tissu osseux est limitée, en comparaison avec les stents notamment. D'autre part, ils présentent également les inconvénients d'être coûteux et de ne pas répondre non plus au problème majeur des fuites de ciment.

Le problème de fuite de ciment a déjà été identifié, notamment dans les documents EP1408888, EP1509175 ou WO200394805 qui expriment l'intérêt que présenterait un implant déformable peu perméable ou imperméable pour limiter ou éviter les fuites de ciment. Ces documents envisagent de nombreuses solutions pour un implant expansible, en métal ou en polymère, qui pourrait être soit souple et flexible comme une membrane ou un tissu, voire élastique, soit semi-rigide (« conformable ») ou rigide, soit en matériau à mémoire de forme, avec une paroi continue ou fenestrée (i.e., maillée) et qui pourrait être poreux ou non poreux. Cependant, toutes ces hypothèses décrites dans ces documents définissent surtout des méthodes envisageables de traitement et des objectifs à atteindre, sans fournir de réel enseignement en ce qui concerne les caractéristiques techniques, ou l'agencement structurel des implants, ni sur la façon d'obtenir de tels implants et ainsi mettre en oeuvre ces méthodes. Ces propositions présentent donc un problème majeur de faisabilité technique.

D'autre part, un problème qui n'est pas identifié dans l'art antérieur concerne du site d'injection du ciment et la répartition des forces exercées sur les tissus osseux pour les redresser. En effet, l'imperméabilité d'un implant permet d'éviter les fuites de ciment, mais la nature de la membrane imperméable et ses caractéristiques techniques comme ses propriétés physico-chimiques et mécaniques influent sur sa capacité à se déployer sans se rompre et à redresser la structure osseuse. Ainsi, une membrane élastique présente l'inconvénient de se déformer excessivement dans les zones à faible densité et d'avoir ainsi une capacité limitée à restaurer une hauteur, avec en plus le risque de se rompre aux endroits où son élasticité maximale est dépassée à cause de cette déformation incontrôlée. Une membrane semi-rigide est donc préférable, mais ce problème de déformation implique également un problème de formes de l'implant, en configuration repliée et surtout en configuration déployée. En effet, la forme de l'implant déployé délimite le site d'injection du ciment et la maîtrise de ce site est importante pour la répartition des forces conduisant à remplir les zones à faible densité tout en redressant la structure (notamment en hauteur), ce qui impacte la réussite de l'opération. Ainsi, on comprend que la fourniture d'un implant répondant à l'ensemble de ces problèmes s'accompagne d'un problème de faisabilité technique et donc de fabrication.

D'autres problèmes récurrents en chirurgie orthopédique concernent l'invasivité (c'est-à-dire le but de faire une incision et des lésions les moins étendues possibles) mais également le ratio de déploiement afin d'obtenir un implant déployé qui comble le plus grand volume possible tout en ayant été introduit par un passage le plus petit possible. D'autre part, ce ratio de déploiement va impacter la répartition des forces pour redresser les vertèbres : si on est trop déformable, la pression d'injection du ciment va plutôt déformer la poche au lieu de restaurer la hauteur.

Un problème complémentaire à celui du déploiement concerne le repliement qui n'est généralement pas possible dans les implants de l'art antérieur. La maîtrise du repliement permet la maîtrise du déploiement et donc du site d'injection avec une répartition homogène du ciment et de la pression pour remplir l'espace à combler suite à l'affaissement. Homothétie parfaite s'adaptant à la fracture en respectant la forme de l'os à l'intérieur de la fracture.

Dans ce contexte, on comprend qu'il persiste dans le domaine un problème technique concernant la restauration de structure osseuse (redressement ou réduction de fracture ou augmentation de volume après affaissement) grâce à un implant expansible (déployable) qui soit capable d'expandre les tissus osseux affaissés et suffisamment imperméable pour éviter ou limiter la fuite de ciment en-dehors de l'implant avec une maîtrise du site d'injection.

Enfin, un problème principal qui persiste dans le domaine concerne la faisabilité technique de la fabrication des implants proposés dans l'art antérieur. Il est par exemple connu le document WO200394805 décrit de nombreuses méthodes d'administration de substances et notamment de ciment osseux, avec de nombreuses variantes envisagées pour un implant expansible, en métal ou en polymère, qui pourrait être soit souple et flexible comme une membrane ou un tissu, soit semi-conformable ou rigide, soit en matériau à mémoire de forme, avec une paroi continue ou fenestrée (i.e., maillée) et qui pourrait être poreux ou non poreux. Cependant, ce document ne décrit que des méthodes envisageables de traitement, mais ne fournit aucun enseignement en ce qui concerne les caractéristiques techniques, ou l'agencement structurel de ces nombreux implants hypothétiques utilisés pour ces méthodes envisagées, ni sur la façon d'obtenir de tels implants et ainsi réellement mettre en oeuvre ces méthodes. Ces propositions présentent donc un problème majeur de faisabilité technique et définissent plus des buts à atteindre que des moyens d'y parvenir. De plus, même si de nombreux objectifs ont été détaillés dans la littérature, de nombreux implants proposés pour atteindre ces objectifs n'ont jamais vu le jour à cause de problèmes de fabrication. Pour répondre au problème de fabrication, il est nécessaire de prendre en compte les problèmes liés à au souhait de compacter / replier un « sac »(ballon/poche) en matériau rigide et imperméable pour :
- passer dans un conduit cylindrique ;
- rendre possible l'expansion sans rupture de la poche, malgré la rigidité et la différence de volume souhaité entre le volume replié et le volume déployé ;
- Maîtriser le volume et la répartition des forces d'expansion sur l'os.

Dans ce contexte, un but de la présente invention est de pallier au moins certains inconvénients de l'art antérieur en proposant un implant de restauration de structure osseuses affaissées fiable et simple à manipuler et implanter.

Ce but est atteint par un implant osseux expansible de chirurgie orthopédique vétérinaire pour la restauration de volume et/ou géométrie d'un os, par une expansion entre une configuration repliée et une configuration déployée, ledit implant s'étendant selon un axe longitudinal entre une extrémité proximale apte à coopérer avec un instrument d'implantation pour tenir l'implant et une extrémité distale destinée à être insérée en premier dans l'os, au moins deux faces de l'implant, par exemple supérieure et inférieure, comportant chacune un plateau pour le contact avec les tissus osseux, chacun des plateaux comportant une portion centrale reliée, par l'intermédiaire d'au moins une charnière, à au moins une paire de bras de support orientés chacun dans des directions opposées au sein de chaque paire, un bras de chaque paire étant relié par une charnière à l'extrémité distale tandis que l'autre bras est relié par une charnière à l'extrémité proximale, l'implant étant apte à recevoir ou comportant un axe central s'étendant au travers d'un manchon de coulissement à l'extrémité proximale jusqu'à une bague ou une douille de traction à l'extrémité distale où il est apte à transmettre une traction, lors de son actionnement par un instrument, sur l'extrémité distale pour permettre de la rapprocher de l'extrémité proximale, en engendrant le pivotement des bras de support provoquant l'éloignement des plateaux l'un par rapport à l'autre et, par conséquent, l'expansion de l'implant entre la configuration repliée et la configuration déployée
caractérisé en ce que :
- au moins deux autres faces de l'implant, entre celles comportant les plateaux, sont couvertes d'au moins une feuille par face, en alliage biocompatible de métaux, et solidarisée de manière étanche aux portions centrales sous les plateaux, à des faces latérales des bras et à des faces latérales de l'extrémité proximale et de l'extrémité distale,
- ladite feuille est déformable plastiquement pour permettre l'expansion de l'implant et présente, au moins dans la configuration repliée, une pluralité de plis antiformes, dit convexes, et synformes, dit concaves, lesdits plis étant couchés les uns sur les autres en configuration repliée, la surface totale de ladite feuille étant supérieure ou égale à la surface latérale de l'implant en configuration déployée de façon à former un compartiment étanche apte à accueillir un fluide à l'intérieur de la cavité obtenue par l'expansion de l'implant

Selon une autre particularité, la distance entre un pli synforme et le pli antiforme suivant est plus longue que la distance entre un pli antiforme et le pli synforme suivant, pour faciliter le repliement de ladite feuille sur la surface des faces latérales de l'implant en configuration repliée.

Selon une autre particularité, ladite feuille de chaque face latérale de l'implant possède, en position déployée, une forme sensiblement de losange, avec une persistance permanente d'au moins une partie des plis repliés à proximité des extrémités proximale et distale.

Selon une autre particularité, ladite feuille est déformable plastiquement également de la configuration repliée à la configuration déployée, notamment grâce à la persistance des plis couchés aux extrémités proximale et distale, facilitant la réversibilité de l'expansion.

Selon une autre particularité, ledit axe central est apte à coopérer avec et/ou se prolonge au-delà de l'extrémité distale d'un instrument d'implantation au niveau de l'extrémité proximale de l'implant et possédant un conduit intérieur en communication avec un conduit ménagé à l'intérieur dudit axe central et débouchant dans l'espace ménagé par l'écartement des plateaux, par au moins une ouverture permettant l'injection dudit fluide dans l'implant.

Selon une autre particularité, solidarisée à une face latérale de l'extrémité proximale par une soudure figeant l'extrémité proximale des plis couchés et repliés contre la paroi extérieure dudit manchon et/ou solidarisée à une face de l'extrémité distale par une soudure figeant l'extrémité distale des plis couchés et repliés contre la paroi extérieure de ladite douille.

Selon une autre particularité, les plis sont, au moins dans la configuration repliée, parallèles à l'axe longitudinal.

Selon une autre particularité, le nombre de plis est compris entre 4 et 16, généralement 6 à 12, de préférence de l'ordre de 8.

Selon une autre particularité, la feuille possède une épaisseur comprise entre 3 et 100 microns, généralement entre 6 et 50 et de préférence 10 et 30 microns.

Selon une autre particularité, la feuille est en alliage de titane.

Selon une autre particularité, distance entre les plis est variable d'une face latérale à l'autre de l'implant, de sorte que la forme de l'implant en configuration déployée soit asymétrique transversalement à l'axe longitudinal.

Un autre but de la présente demande est de pallier au moins une partie des inconvénients de l'art antérieur en proposer un système d'intervention chirurgicale facile à utiliser et permettant une stabilisation efficace des tissus osseux

Ce but est atteint par un système de traitement orthopédique de tissu osseux lésé comprenant un ciment de substitution osseuse et au moins un instrument d'implantation et d'injection de ciment dans l'implant, caractérisé en ce qu'il comporte un implant selon l'un des modes de réalisation décrits ici.

Selon une autre particularité, l'instrument d'implantation et d'injection de ciment comporte des moyens de contrôle de la pression et/ou de l'aspiration du ciment pour replier l'implant en configuration repliée si nécessaire.

Selon une autre particularité, l'instrument d'implantation est distinct mais complémentaire de l'instrument d'injection dont le canal d'injection du ciment traverse un canal à l'intérieur d'une tige creuse de l'instrument d'implantation configurée pour tenir l'extrémité proximale de l'implant à l'extrémité distale de ladite tige creuse.

Un autre but de la présente demande est de pallier au moins une partie des inconvénients de l'art antérieur en proposer un procédé fabrication d'un implant d'intervention chirurgicale facile à utiliser et permettant une stabilisation efficace des tissus osseux

Ce but est atteint par un procédé de fabrication d'un implant selon l'invention, caractérisé en ce qu'il comporte :
Obtention d'un implant expansible à deux plateaux s'écartant sous l'effet du rapprochement des extrémités de l'implant grâce à des bras supports reliant ces extrémités aux plateaux ;
Insertion d'une feuille de matériau biocompatible métallique entre deux crémaillères munies des rainures de profil triangulaire pour imprimer des plis à la feuille ;
Repliement des plis les uns sur les autres, pour obtenir une feuille pliée ;
Disposition de ladite feuille pliée sur une face latérale de l'implant ;
Solidarisation de ladite feuille sur ladite face latérale ;
Réitération des étapes b) à e) pour l'autre face de l'implant.

D'autres particularités et avantages de la présente invention apparaîtront plus clairement à la lecture de la description de divers modes de réalisation ci-après, faite en référence aux dessins annexés, dans lesquels :

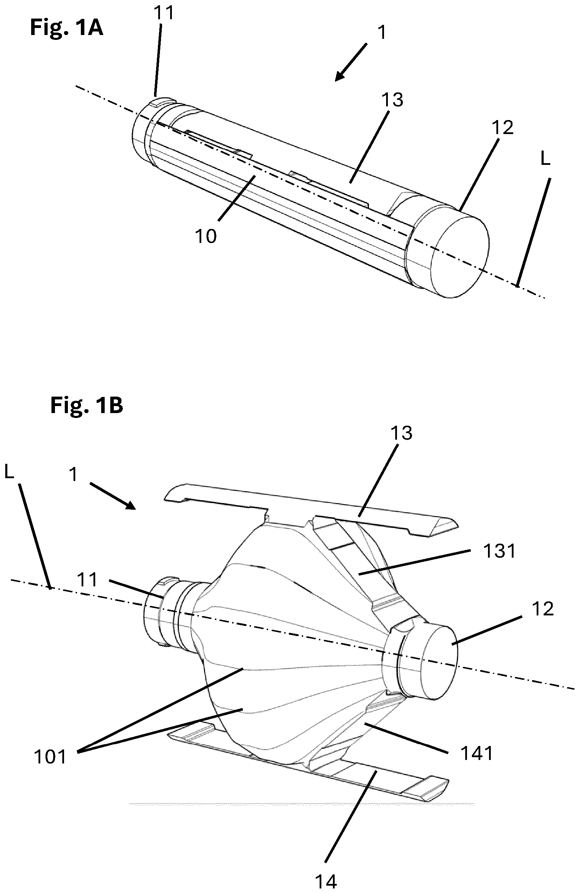

Figures 1 : La figure 1A représente une vue en perspective d'un implant expansible dans sa configuration repliée, selon certains modes de réalisation, et la figure 1B représente une vue en perspective de ce même implant en configuration déployée ;

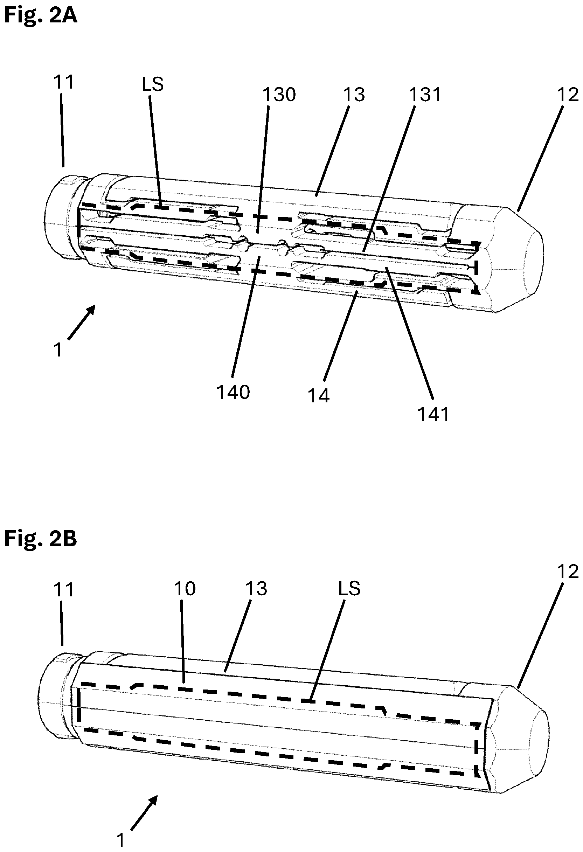

Figures 2 : La figure 2A représente une vue en perspective d'un implant expansible duquel a été retiré la feuille et représentant la ligne de soudure de cette feuille et la figure 2B représente le même implant dans laquelle la feuille est présente et la ligne de soudure est représentée en pointillée ;

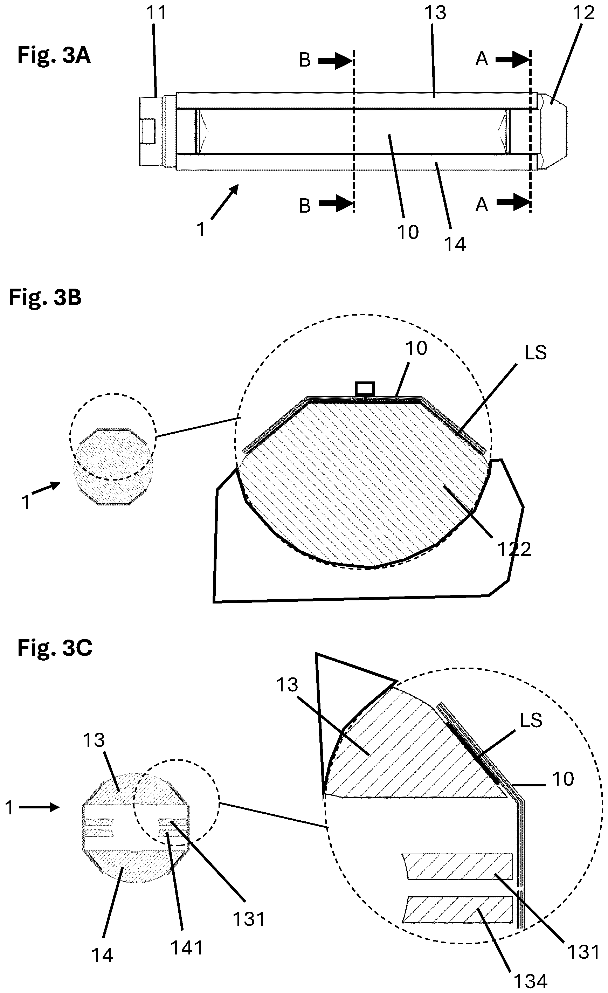

Figures 3 : la figure 3A représente une vue en transparence d'un implant expansible selon certains modes de réalisation, avec les plans de coupe BB et CC des figures respectivement 3B et 3C qui représente des vues en coupe, respectivement selon les plans de coupe BB et CC de la figure A et chacune un agrandissement montrant la ligne de soudure de la feuille sur l'implant dans ces plans de coupe, selon certains modes de réalisation ;

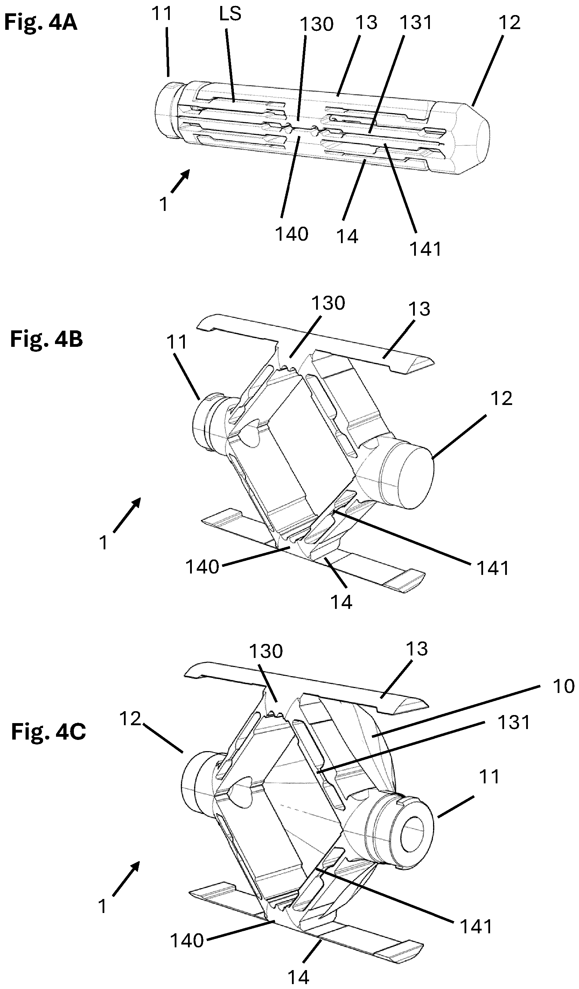

Figures 4 : la figure 4A représente une vue en perspective d'un implant expansible en configuration repliée et dépourvu de sa feuille, selon certains modes de réalisation, et la figure 4B représente le même implant que celui de la figure 4A mais en configuration déployée et la figure 4C représente un implant expansible muni d'une feuille sur seulement l'une de ses faces ;

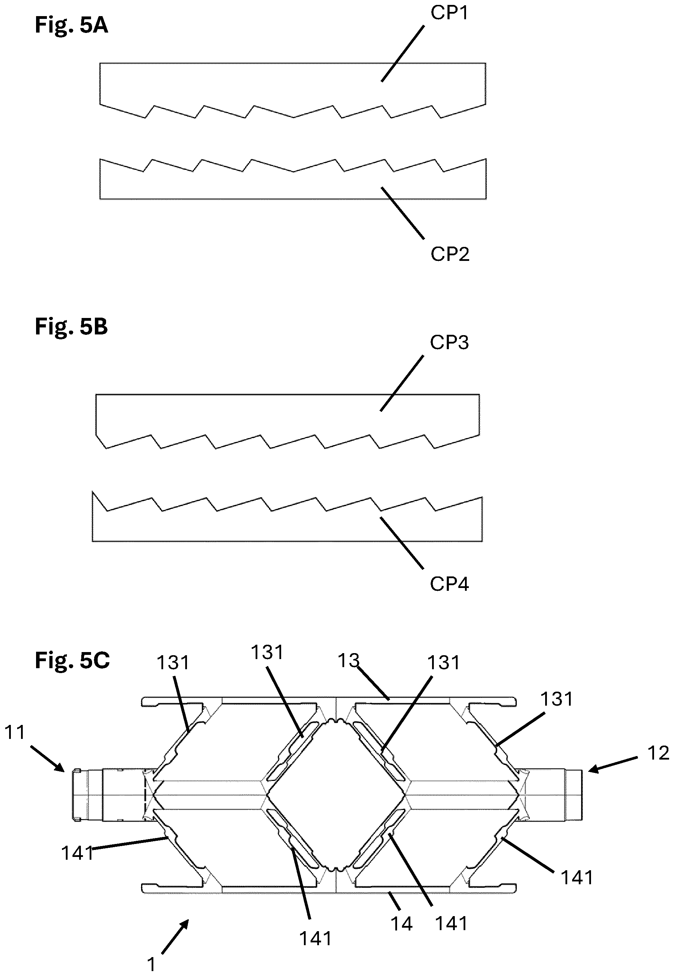

Figures 5 : la figure 5A représente une vue de profil d'une crémaillère de pré-pliage de feuilles pour implants expansibles selon certains modes de réalisation et la figure 5B représentent une vie de profil d'une crémaillère de pré-pliages selon d'autres modes de réalisation et la figure 5C représente une vue de profil d'un implant expansible à double bras de support selon certains modes de réalisation ;

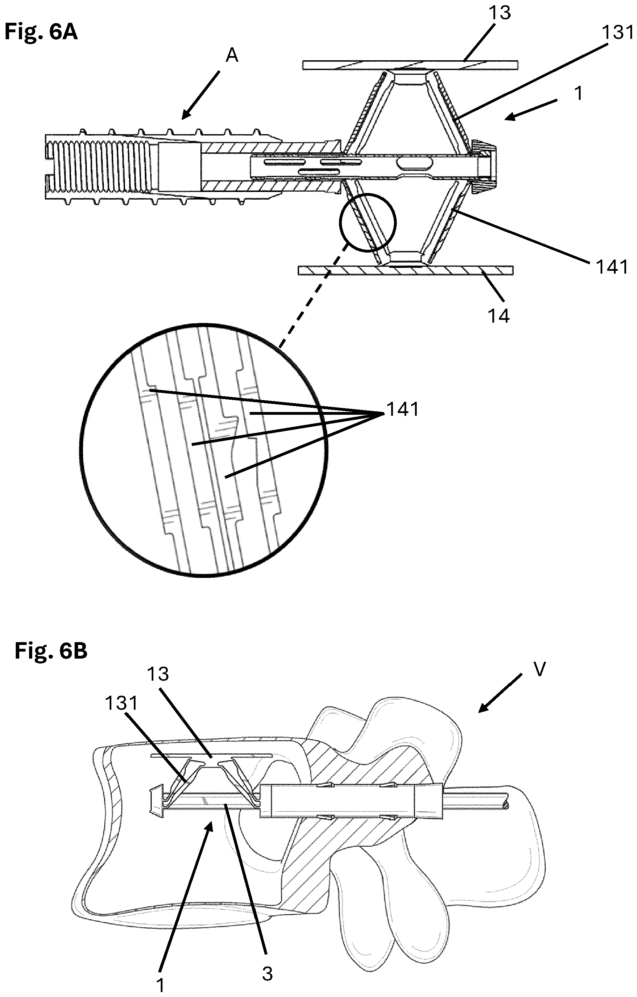

Figures 6 : la figure 6A représente une vue en coupe d'un implant expansible porté par un instrument d'implantation et avec un agrandissement montrant des détails des bras de supports doubles avec un mécanisme d'auto-verrouillage, la figure 6B représente une vue en coupe d'une vertèbre dans laquelle est implanté un implant expansible selon d'autres modes de réalisation ;

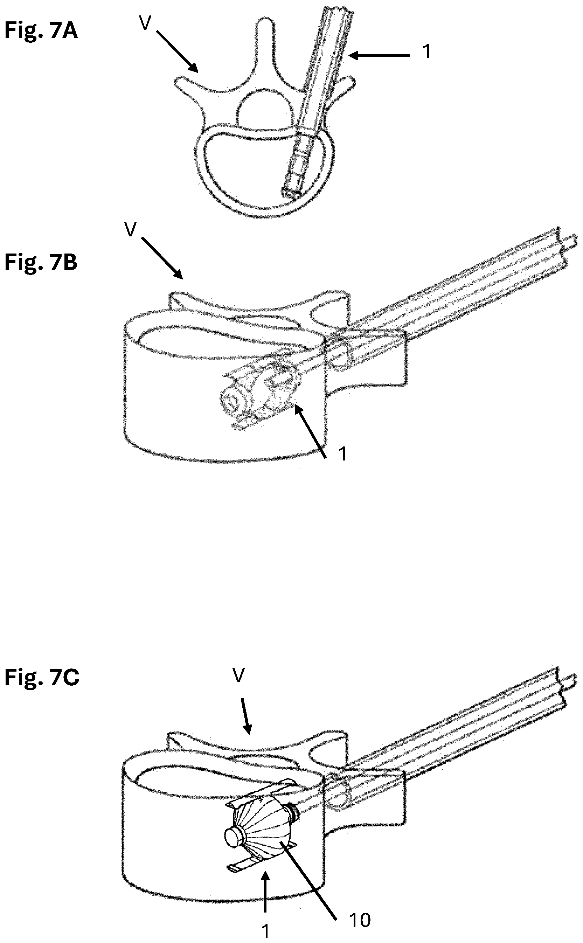

Figures 7 : la figure 7A représente une vue de dessus d'une vertèbre dans laquelle est implanté un implant selon divers modes de réalisation, la figure 7B représente une vue en perspective d'une vertèbre dans laquelle est implanté un implant de l'art antérieur et la figure 7C représente une vue en perspective d'une vertèbre dans laquelle est implanté un implant selon certains modes de réalisation ;

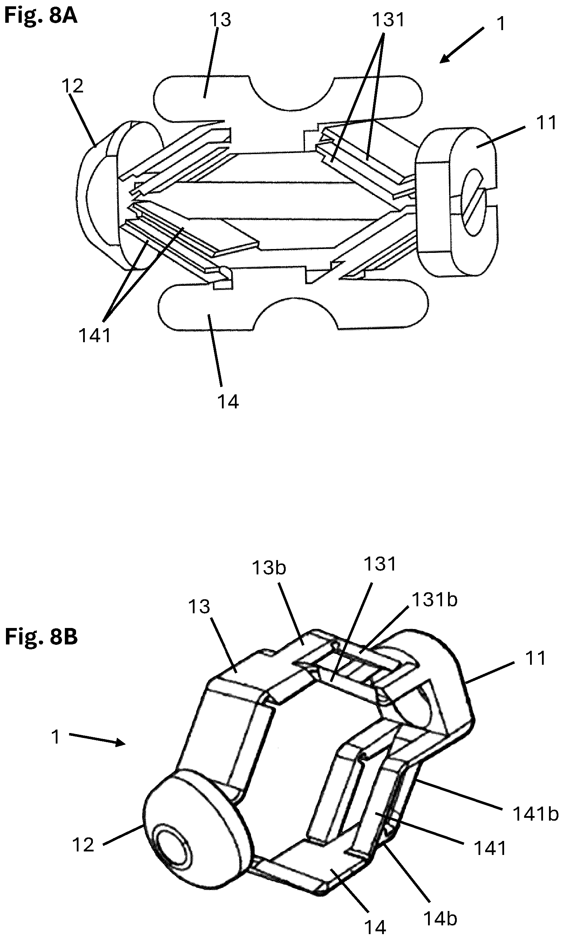

Figures 8 : la figure 8A représente une vue en perspective d'un implant selon certains modes de réalisation et la figure 8B représente une vue en perspective d'un implant selon d'autres modes de réalisation ;

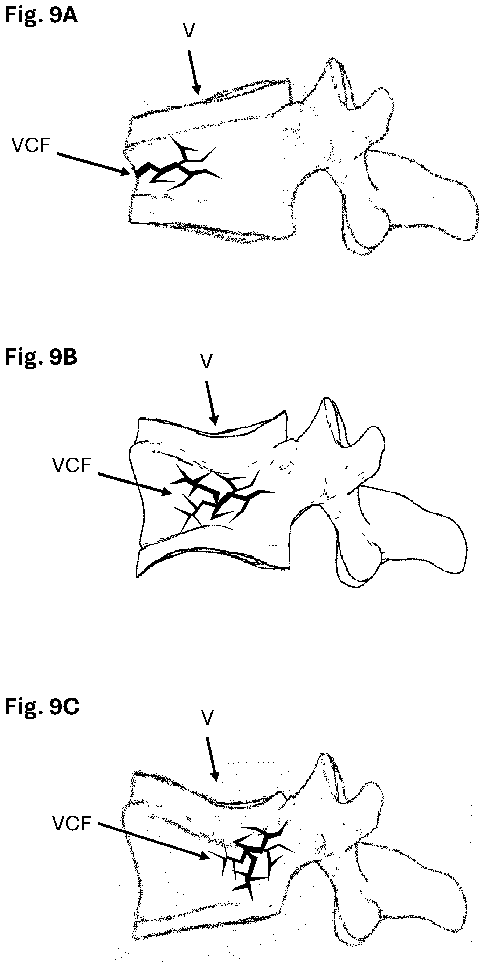

Figures 9 : Les figures 9A, 9B et 9C représentent des vues de profil de vertèbres ayant subi des fractures de compression vertébrale (VCF) respectivement au niveau antérieur, médian et postérieur ;

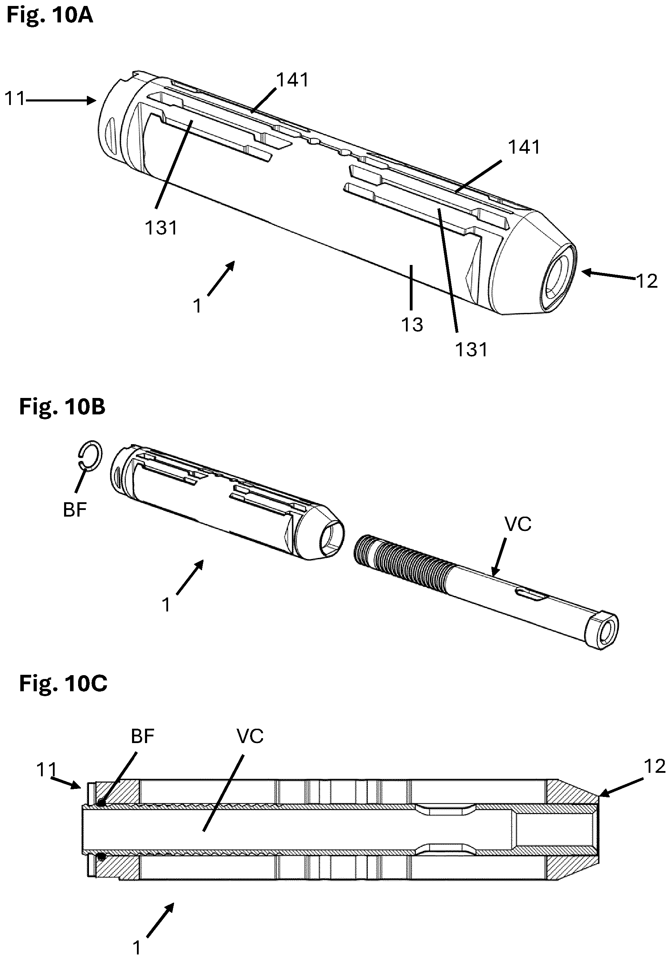

Figures 10 : La figure 10A représente une vue en perspective d'un implant dépourvu de sa feuille selon certains modes de réalisation et la figure 10B représente ce même implant duquel a été retiré la tige d'expansion et la figure 10C représente une vue en coupe de l'implant de la figure 10A dans lequel est présente la tige d'expansion.

La présente demande concerne un implant et un système de chirurgie orthopédique pour le traitement d'os fracturé et de tissus osseux en général, ainsi qu'un procédé de fabrication de l'implant. L'implant osseux est de préférence un implant Rachidien, et en particulier vertébral ou même en fait intravertébral, mais d'autres utilisations sont envisageables ailleurs dans le rachis (épines intervertébrales) ou dans d'autres structures osseuses où il est nécessaire de combler un espace laissé résultant d'une fracture (dont les causes peuvent être diverses, même si elles impliquent généralement une diminution de densité osseuse). Ainsi, les fractures vertébrales de compression (VCF pour l'anglais Vertébral Compression Fracture) sont une application favorite mais ne sont pas les seules à pouvoir être traitées grâce à la présente invention et l'homme de métier appréciera les possibilités offertes sans nécessiter plus de détails ici. Comme autre os, on peut citer le fémur ou l'humérus (tête) par exemple en cas de risque d'effondrement. La présente demande concerne un implant et un système de chirurgie orthopédique vétérinaire pour le traitement d'os fracturé et de tissus osseux en général, ainsi qu'un procédé de fabrication de l'implant. L'implant osseux est de préférence un implant Rachidien, et en particulier vertébral ou même en fait intravertébral, mais d'autres utilisations sont envisageables ailleurs dans le rachis (épines intervertébrales) ou dans d'autres structures osseuses où il est nécessaire de combler un espace laissé résultant d'une fracture (dont les causes peuvent être diverses, même si elles impliquent généralement une diminution de densité osseuse). Ainsi, les fractures vertébrales de compression (VCF pour l'anglais Vertébral Compression Fracture) sont une application favorite mais ne sont pas les seules à pouvoir être traitées grâce à la présente invention et l'homme de métier appréciera les possibilités offertes sans nécessiter plus de détails ici. Comme autre os, on peut citer le fémur ou l'humérus (tête) par exemple en cas de risque d'effondrement. Dans le domaine vétérinaire, il est connu que les animaux ont des densités osseuses parfois très différentes de l'homme et surtout très variables selon les espèces et même selon les races ou animaux au sein d'une même espèces, notamment pour les chiens dont les propriétés physiques varient énormément d'une race à l'autre. Par exemple, les chiens de type teckel ou similaires ont des vertèbres longues (hautes) mais peu larges comparé aux autres espèces. Il est donc utile de disposer d'implants expansibles qui permettent une forte expansion en hauteur tout en ayant une longueur réduite et il est clairement nécessaire de tenir compte des différences de formes et dimensions des os des différentes espèces pour adapter la thérapie efficacement avec des implants adaptés. De plus, certaines espèces comme le chat ont une corticale osseuse très rigide mais du tissu spongieux plus souple que d'autres espèces. Il est donc nécessaire de prendre également la nature des tissus osseux. Enfin, un autre exemple notable concerne les chevaux qui ont des os, notamment les vertèbres, avec une forme anatomique particulière et qui supportent parfois une charge importante. Selon l'activité (par exemple sportive) et la morphologie, la densité osseuse varie et les implants doivent être adaptés pour permettre leur expansion mais aussi supporter les charges. Ainsi, pour un cheval, il peut être nécessaire d'avoir des implants comprenant plus de bras de support de charge (minimum 3 ou 4) que pour d'autres espèces (où 2 bras support suffisent parfois). En l'absence de bras de support, il est alors nécessaire que l'implant ait une résistance mécanique suffisante, grâce à une capacité à supporter une pression de ciment supérieure à d'autres cas.

Certains modes de réalisation prévoient l'injection d'un fluide (e.g., « ciment osseux », généralement à base d'un polymère comme le PMMA par exemple et bien connu de l'homme de métier, de sorte qu'aucun détails sur le ciment ne sera fourni ici). Ainsi, l'implant une fois positionné peut être stabilisé grâce une telle injection de ciment. Cependant, comme les fuites de ciment sont un problème majeur dans le domaine, divers modes de réalisation proposent de contenir le ciment dans une enveloppe étanche dont le volume après injection peut être maîtrisé grâce à la structure et le matériau de l'enveloppe, en fonction de la pression injectée (et de la configuration des tissus osseux préférentiellement évaluée à l'avance, comme généralement pratiqué dans le domaine). L'étanchéité est bien entendu relative et ce terme n'est pas limitatif non plus, puisque le niveau d'étanchéité est en fait adapté à la viscosité du ciment au moment où on l'injecte. Certains modes de réalisation permettent en particulier un gonflement homothétique de l'enveloppe grâce à la souplesse (relative) de la feuille (10) de matériau métallique biocompatible. Ce matériau est en général un alliage de titane obtenu sous forme de feuille très fine, de préférence par lamination pour un état de surface et une épaisseur maîtrisés, notamment une épaisseur comprise entre 3 et 100 microns, généralement entre 6 et 50 et de préférence 10 et 30 microns. Cette feuille est capable de déformation plastique réversible un nombre de fois satisfaisant pour l'application visée puisqu'elle offre notamment la possibilité de rétracter l'enveloppe formée par la feuille en cas de problème (biocompatibilité et résistance au déchirement). En effet, en général, le pilotage du dosage de ciment permet de surveiller les quinze minutes de polymérisation pendant lesquelles il est possible de rétracter l'enveloppe et aspirer le ciment. D'autre part, par l'injection de ciment, le gonflement de l'enveloppe, l'implant remplit les espaces dans les tissus lésés en fonction des forces de compression et de résistance osseuse par rapport à la pression hydraulique fournie lors de l'injection de ciment. A partir d'une telle feuille, il est nécessaire d'obtenir une structure fermée, ce qui impose déjà de refermer la feuille sur elle-même et de la verrouiller en position. Pour cela, une soudure (ou un collage ou une brasure, ces termes n'étant pas limitatifs ici) peut être réalisée entre deux bords qui se superposent ou sur des bords avec des revers enchevêtrés, pour faciliter et solidifier la soudure. Certains modes de réalisation prévoient donc une fermeture par soudure depuis l'extérieur, simplifié et plus solide grâce à la superposition de couches au niveau de ces plis complémentaires.

Divers modes de réalisation permettent d'obtenir un implant expansible de dimensions très réduite en configuration repliée tout en garantissant un volume satisfaisant en configuration déployée. Ainsi, le passage nécessaire à l'introduction des implants de la présente demande est généralement inférieur à celui des implants connus alors que l'expansion est supérieure à celle de ces implants connus. En effet, le diamètre ou le volume replié est inférieur au diamètre déployé d'un facteur compris entre 3 et 20, généralement 3 à 8, de préférence 4 à 7. Ce rapport dépend bien entendu de la quantité de ciment injecté et certains modes de réalisation tirent avantage du fait de pouvoir prévoir un implant capable de se déployer plus que nécessaire, notamment en conservant des plis en configuration déployée. Ainsi, la détermination du volume de l'implant sera effectuée en se basant sur la taille réduite nécessaire à l'introduction dans les tissus osseux et donc en référence au volume replié. Cependant, différents volumes sont prévus pour la configuration déployée, puisque le nombre de plis et la longueur des plis permet d'augmenter le ratio de déploiement.

Le terme « solidarisé » signifie ici que deux éléments sont rendus solidaires, soit de manière permanente (ou quasi-permanente) mais également parfois qu'une liaison est réalisée pour l'actionnement d'un élément par un autre. Ainsi, un vissage ou une coopération de forme pour verrouiller provisoirement les éléments entre sont couverts par ce terme non limitatif.

Les termes bague, manchon, ou tube désignent des structures creuses telles que des anneaux, des conduits ou des tuyaux mais de façon non limitative, notamment avec des formes diverses (à l'intérieur comme à l'extérieur) bien que la forme cylindrique soit préférée. Le terme canal est en revanche de préférence utilisé ici pour désigner un passage plutôt que l'élément qui le contient et le terme ouverture désigne ici le fait qu'un élément soit ouvert et apte à être traversé en débouchant dans une autre structure ou un autre élément. Généralement les termes manchon et tubes ou conduits désignent des éléments plus longs que les bagues ou anneaux, mais leur utilisation ici n'est pas limitative non plus. D'autre part, les termes douille ou culot désignent également des structures creuses qui sont ouvertes à une extrémité mais fermées à l'autre extrémité, comme des bouchons, des fermetures, des constrictions ou étranglement et ces termes sont utilisés indifféremment sans limitation quelconque.

Le terme « charnière » est utilisé ici dans son acception fonctionnelle, sans impliquer de limitation structurelle et peut désigner en fait des charnières mécaniques, même si elles se de préférence formées (comme illustrés sur les exemples non limitatifs des figures), par des amincissement (ou rétrécissement, retrait de matière) des éléments tels que les bras de support ou autre. Ainsi, une charnière est en fait un point ou une zone d'articulation, puisqu'il est connu dans le domaine qu'il est généralement sans danger de prévoir de tels mécanismes de pivotement pour des implants car les matériaux qui les composent sont adaptés à ce type d'articulation.

Les termes pli antiforme, dit convexe, et un pli synforme, dit concave son utilisés ara analogie avec les définitions de plis dans de nombreux domaines techniques, dont la géologie, mais on comprendra que la convexité est ici définie par rapport à l'extérieur de l'implant. Un pli antiforme ou convexe est donc un pli qui rabat la matière vers l'intérieur, tandis qu'un plis antiforme rabat la matière vers l'extérieur. La succession des deux types de plis permet de limiter un maximum le volume replié. De plus, certains modes de réalisation prévoient des plis longs et des plis courts se succédant pour faciliter l'enroulement et/ou le tassement, en limitant la superposition de matière en configuration repliée. On notera également que le nombre de plis n'est pas limitatif non plus et qu'il permet en revanche de conserver l'irrégularité ou la réalité de forme de l'implant lors du déploiement, ce qui présente également des avantages, notamment en termes de stabilisation. De plus, il reste préférable de prévoir un équi-répartition des surfaces entre les plis pour un déploiement uniforme permettre un déploiement uniforme mais l'invention envisage également d'autres applications et notamment des plis de différentes tailles selon la région de l'implant, afin d'obtenir un déploiement asymétrique et de meilleurs résultats thérapeutique. Par ailleurs, la présente invention permet de maîtriser la forme de l'implant une fois déployé en prévoyant également la distance entre les plis. En effet, la distance entre les plis synforme/antiforme et donc la distance entre les plis longs et les plis courts conditionne la façon de se déployer. Avantageusement, si la densité est plus dense à un endroit de la périphérie, le déploiement sera plus important et si elle est moins dense, l'enveloppe pourra moins se déployer. On comprend que l'obtient ainsi une asymétrie et une incurvation par un déploiement plus étendu dans les zones présentant le plus de plis. De même, il est possible de prévoir plus de matière (une grande surface de la feuille d'un côté par exemple, pour que l'expansion latérale soit plus importante de ce côté que de l'autre. Par ailleurs, dans certains modes de réalisation, la feuille est soudée aux plateaux et ne peut donc pas se déployer plus loin que la distance entre les plateaux, qui aura été réglé par le mécanisme de levée. On obtient ainsi un implant dont l'expansion est limitée dans une dimension (la dimension essentielle en générale, où l'on souhaite restaurer une hauteur ou largeur précise), mais pas dans une autre dimension, de sorte que l'injection de ciment déploiera l'enveloppe dans les volumes d faible densité osseuse éventuellement présentes autour de l'implant. On notera d'ailleurs que l'instrument d'injection de fluide (Ac) peut être muni de moyens de contrôler la pression injectée (un manomètre par exemple) et de savoir quel est le volume résultant, afin de contrôler efficacement l'expansion dans les tissus osseux. Enfin, on comprend que l'instrumentation proposée dans la présente demande dans certains modes de réalisation, en utilisant un porte-implant (ou ancillaire) relativement classique pour tenir l'implant et l'introduire dans les tissus osseux, mais également moins classique pour l'expandre dans les tissus osseux, présente également l'avantage de pouvoir réaliser toutes les étapes d'implantation et de stabilisation avec un seul instrument et en une opération continue. En effet, l'ancillaire avec un tube creux d'acheminement du ciment au travers du tube qui retient le ciment, permet de disposer d'un instrument permettant d'effectuer l'opération chirurgicale rapidement et efficacement. Après le perçage, on introduit l'implant et sans retirer l'instrument, on peut gonfler l'enveloppe avec le ciment et ensuite retirer l'outil avant, pendant ou même après la polymérisation du ciment (par exemple à l'aide d'un mécanisme de coupe du ciment dur lors d'une rotation de l'instrument). Le temps d l'opération chirurgicale est bien entendu nettement diminué mais également la stabilité de l'implant qui n'est lâché à aucun moment tant qu'il n'est pas stabilisé par l'injection de ciment remplissant tous les volumes libres autour, contrairement à certaines solutions de l'art antérieur.

Les termes « cylindre », « cylindrique » ou « cylindre généralisé » sont utilisés dans la présente demande sans distinction pour faciliter l'exposé de l'invention et désignent en fait tous un « cylindre généralisé » c'est-à-dire une forme tridimensionnelle définie par une hauteur (parallèle à l'axe longitudinal) et deux bases (transversales à l'axe longitudinal) qui peuvent avoir n'importe quelle forme, même si la forme circulaire est préférée pour simplifier la fabrication et limiter le risque de lésion des tissus dans lesquels elle est introduite. De préférence, ce « cylindre » est droit, c'est-à-dire que ses bases sont alignées selon la génératrice (ou hauteur) du cylindre. D'autre part, comme l'implant peut se déployer dans un tissu en se conformant à la forme de l'espace dans lequel il est introduit (en le modifiant grâce à la pression qu'il exerce sur ces issus), la forme peut ne pas être constante et les deux bases du cylindre peuvent avoir des formes (surfaces) différentes.

Par conséquent, le terme « diamètre » est utilisé, dans la présente demande, pour désigner en fait la plus grande dimension du cylindre généralisé transversalement à la hauteur (ou axe longitudinal), c'est-à-dire dans un plan (dit « transversal ») parallèle à celui des bases d'un tel cylindre généralisé. Ainsi, le terme diamètre peut en fait désigner la longueur de la diagonale d'un carré ou d'un rectangle ou encore (pour une forme quelconque) la plus grande distance entre deux points inclus dans un tel plan transversal et situés sur la circonférence d'un tel cylindre. De même, les termes « circonférence », « périphérie » ou « périmètre » sont utilisés ici pour désigner le pourtour de ces bases de forme quelconque.

De même, les termes « conique » ou « tronconique » sont utilisés ici pour désigner des formes qui s'évasent depuis un « diamètre » (ou une aire/surface) minimum jusqu'à un « diamètre » maximum, mais ils n'impliquent aucune limitation sur la forme de la périphérie qui peut être circulaire ou non.

D'une manière générale, la présente demande concerne un implant (1) osseux expansible de chirurgie orthopédique vétérinaire pour la restauration de volume et/ou géométrie d'un os, par une expansion entre une configuration repliée et une configuration déployée, ledit implant s'étendant selon un axe longitudinal (L) entre une extrémité proximale (11) apte à coopérer avec un instrument d'implantation (A) pour tenir l'implant et une extrémité distale (12) destinée à être insérée en premier dans l'os, au moins deux faces de l'implant, par exemple supérieure et inférieure, comportant chacune un plateau (13, 14) pour le contact avec les tissus osseux, chacun des plateaux comportant une portion centrale (130, 140) reliée, par l'intermédiaire d'au moins une charnière, à au moins une paire de bras de support (131, 141) orientés chacun dans des directions opposées au sein de chaque paire, un bras de chaque paire étant relié par une charnière à l'extrémité distale (11) tandis que l'autre bras est relié par une charnière à l'extrémité proximale (12), l'implant (1) étant apte à recevoir ou comportant un axe (3) central s'étendant au travers d'un manchon de coulissement à l'extrémité proximale (11) jusqu'à une bague ou une douille de traction à l'extrémité distale (12) où il est apte à transmettre une traction, lors de son actionnement par un instrument (A), sur l'extrémité distale (12) pour permettre de la rapprocher de l'extrémité proximale (11), en engendrant le pivotement des bras de support (131, 141) provoquant l'éloignement des plateaux (13, 14) l'un par rapport à l'autre et, par conséquent, l'expansion de l'implant entre la configuration repliée et la configuration déployée caractérisé en ce que :
- au moins deux autres faces de l'implant, entre celles comportant les plateaux, sont couvertes d'au moins une feuille (10) par face, en alliage biocompatible de métaux, et solidarisée de manière étanche aux portions centrales (130, 140) sous les plateaux, à des faces latérales des bras (131, 141) et à des faces latérales de l'extrémité proximale (11) et de l'extrémité distale (12),
- ladite feuille (10) est déformable plastiquement pour permettre l'expansion de l'implant et présente, au moins dans la configuration repliée, une pluralité de plis antiformes (101), dit convexes, et synformes (102), dit concaves, lesdits plis étant couchés les uns sur les autres en configuration repliée, la surface totale de ladite feuille étant supérieure ou égale à la surface latérale de l'implant en configuration déployée de façon à former un compartiment étanche apte à accueillir un fluide à l'intérieur de la cavité obtenue par l'expansion de l'implant

Dans certains modes de réalisation, la distance entre un pli synforme et le pli antiforme suivant est plus longue que la distance entre un pli antiforme et le pli synforme suivant, pour faciliter le repliement de ladite feuille sur la surface des faces latérales de l'implant en configuration repliée.

Dans certains modes de réalisation, ladite feuille de chaque face latérale de l'implant possède, en position déployée, une forme sensiblement de losange, avec une persistance permanente d'au moins une partie des plis repliés à proximité des extrémités proximale (11) et distale (12).

Dans certains modes de réalisation, ladite feuille est déformable plastiquement également de la configuration repliée à la configuration déployée, notamment grâce à la persistance des plis couchés aux extrémités proximale et distale, facilitant la réversibilité de l'expansion.

Dans certains modes de réalisation, ledit axe central (3) est apte à coopérer avec et/ou se prolonge au-delà de l'extrémité distale d'un instrument (A) d'implantation au niveau de l'extrémité proximale de l'implant et possédant un conduit intérieur en communication avec un conduit (31) ménagé à l'intérieur dudit axe (3) central et débouchant dans l'espace ménagé par l'écartement des plateaux, par au moins une ouverture (32) permettant l'injection dudit fluide dans l'implant (1). De façon connu, l'instrument peut comporter une tige d'expansion (A3) montée libre dans l'instrument et permettant d'actionner l'implant en rapprochant l'extrémité distale de l'extrémité proximale et obtenir l'expansion.

Dans certains modes de réalisation, solidarisée à une face latérale de l'extrémité proximale (11) par une soudure (110) figeant l'extrémité proximale des plis couchés et repliés contre la paroi extérieure dudit manchon et/ou solidarisée à une face de l'extrémité distale (12) par une soudure (120) figeant l'extrémité distale des plis couchés et repliés contre la paroi extérieure de ladite douille.

Dans certains modes de réalisation, les plis sont, au moins dans la configuration repliée, parallèles à l'axe longitudinal (L).

Dans certains modes de réalisation, le nombre de plis est compris entre 4 et 16, généralement 6 à 12, de préférence de l'ordre de 8.

Dans certains modes de réalisation, la feuille (10) possède une épaisseur comprise entre 3 et 100 microns, généralement entre 6 et 50 et de préférence 10 et 30 microns.

Dans certains modes de réalisation, la feuille (10) est en alliage de titane.

Dans certains modes de réalisation, distance entre les plis est variable d'une face latérale à l'autre de l'implant, de sorte que la forme de l'implant en configuration déployée soit asymétrique transversalement à l'axe longitudinal (L).

Dans certains modes de réalisation, au moins une partie des bras de support sont prévus en doubles exemplaires. En effet, notamment pour limiter les risques d'expansion non régulière des plateaux, à cause des bras de support en opposition de force et des contraintes extérieures exercées sur l'implant, il est préférable de prévoir des bras support en double exemplaire, par exemple comme représenté sur les figures 6A, 6B, 8A et 8B.

De plus, de tels bras doubles peuvent comporter un mécanisme d'auto-verrouillage en configuration déployée, comme par exemples des crans ménagés face à face de sorte à s'engager entre eux, par exemple comme représenté sur l'encadré agrandi de la figure 6A.

Par ailleurs, pour améliorer encore la fiabilité et la symétrie de l'expansion, il est possible de prévoir des bras de support qui soient déportés vers les extrémités des plateaux au lieu des seuls bras support simples articulés au centre des plateaux, par exemple comme représenté sur la figure 5C. Une telle configuration permet de créer des parallélogrammes déformables qui conservent leur propriété de parallélisme entre leurs côtés, ce qui rend l'expansion plus fiable.

En ce qui concerne l'actionnement et le verrouillage, on notera que les diamètres très fins des implants et leurs axes centraux sont difficilement compatibles avec des filetages pour opérer l'expansion par vissage au niveau de l'implant, alors qu'il est avantageux d'effectuer un vissage au niveau de l'instrument actionnant l'expansion, notamment lorsque l'expansion implique un rapprochement des bras support les uns par rapport aux autres.. Ainsi, comme connu de l'art antérieur, il est possible d'utiliser par exemple une bague fendue logée dans un renforcement circulaire de l'implant et coopérant avec des crans sur l'axe de poussée ou de traction qui sont orientés pour permettre le passage de cet axe dans un seul sens, par exemple comme représenté sur les figures 10A, 10 et 10C. Ainsi, l'actionnement de l'axe pour l'expansion des plateaux peut être réalisé par passage successifs des crans, ce qui permet de former un verrou cranté (VC) pour verrouiller l'implant en configuration déployée.

La présente demande concerne également un système de traitement orthopédique de tissu osseux lésé comprenant un ciment de substitution osseuse et au moins un instrument (A) d'implantation et d'injection de ciment dans l'implant, caractérisé en ce qu'il comporte un implant selon l'un des modes de réalisation décrits ici.

Dans certains modes de réalisation, l'instrument d'implantation et d'injection de ciment comporte des moyens de contrôle de la pression et/ou de l'aspiration du ciment pour replier l'implant en configuration repliée si nécessaire.

Dans certains modes de réalisation, l'instrument d'implantation est distinct mais complémentaire de l'instrument d'injection dont le canal d'injection du ciment traverse un canal à l'intérieur d'une tige creuse de l'instrument d'implantation configurée pour tenir l'extrémité proximale de l'implant à l'extrémité distale de ladite tige creuse.

Dans certains modes de réalisation, l'implant peut comporter une seconde feuille (10) entourant la première feuille, dans le même matériau ou un autre, par exemple pour une isolation thermique protégeant les tissus de la chaleur de la polymérisation. Dans ce cas, les extrémités comportent une bague supplémentaire pour fixer cette seconde feuille en préservant un espace entre celle-ci et la première feuille, avec potentiellement une entrée d'injection d'un autre fluide entre les deux. Ces deux feuilles peuvent alors être pliés et enroulées en même temps lors de la fabrication. Ces modes de réalisation permettent un préformage du site d'injection (par écrasement du tissus osseux spongieux) et permet par exemple d'injecter en deux temps ledit fluide pour un meilleur ajustement de la forme, de la température résultante dans les tissus et/ou de la vitesse de polymérisation du fluide.

La présente demande concerne également un procédé de fabrication d'un implant selon l'une des revendications précédentes, caractérisé en ce qu'il comporte :
- Obtention d'un implant expansible à deux plateaux s'écartant sous l'effet du rapprochement des extrémités de l'implant grâce à des bras supports reliant ces extrémités aux plateaux ;
- Insertion d'une feuille de matériau biocompatible métallique entre deux crémaillères munies des rainures de profil triangulaire pour imprimer des plis à la feuille ;
- Repliement des plis les uns sur les autres, pour obtenir une feuille pliée ;
- Disposition de ladite feuille pliée sur une face latérale de l'implant ;
- Solidarisation de ladite feuille sur ladite face latérale ;
- Réitération des étapes b) à e) pour l'autre face de l'implant.

Par exemple l'enroulement peut être réalisé par l'introduction de la feuille refermée et prépliée dans un conduit dont le diamètre se rétrécit progressivement jusqu' au diamètre souhaité pour l'implant, par coulissement et rotation de l'implant dans ce conduit (par exemple avec un guide à l'intérieur de la feuille pour éviter qu'elle ne s'écrase).

La solidarisation avec le manchon et la douille se fera généralement par soudure (120) et de préférence avec un écrasement préalable de la feuille sur la circonférence de la douille ou le manchon, par exemple par une bague de compression (121) dont des exemples sont représentés sur certaines figures. En effet, il est possible de souder directement mais un écrasement des plis à l'endroit reste préférable.

La présente demande décrit diverses caractéristiques techniques et avantages en référence aux figures et/ou à divers modes de réalisation. L'homme de métier comprendra que les caractéristiques techniques d'un mode de réalisation donné peuvent en fait être combinées avec des caractéristiques d'un autre mode de réalisation à moins que l'inverse ne soit explicitement mentionné ou qu'il ne soit évident que ces caractéristiques sont incompatibles ou que la combinaison ne fournisse pas une solution à au moins un des problèmes techniques mentionnés dans la présente demande. De plus, les caractéristiques techniques décrites dans un mode de réalisation donné peuvent être isolées des autres caractéristiques de ce mode à moins que l'inverse ne soit explicitement mentionné.

### Liste détaillée des références dans les figures :

- 1: implant
- 10: feuille
- 101: pli
- 11: extrémité proximale
- LS: ligne de soudure
- 12: extrémité distale
- A: instrument d'implantation
- A3: tige d'expansion
- CP1: première crémaillère de pré-pliage
- CP2: seconde crémaillère de pré-pliage
- CP3: troisième crémaillère de pré-pliage
- CP4: quatrième crémaillère de pré-pliage
- 3: axe central
- 31: conduit dans l'axe central
- 32: ouvertures du conduit de l'axe central
- 13: premier plateau
- 14: deuxième plateau
- 15: troisième plateau
- 130: portion centrale du premier plateau
- 140: portion centrale du deuxième plateau
- 131: bras support du premier plateau
- 141: bras support du deuxième plateau
- VC: verrou cranté

## Revendications

1. Implant (1) osseux expansible de chirurgie orthopédique vétérinaire pour la restauration de volume et/ou géométrie d'un os, par une expansion entre une configuration repliée et une configuration déployée, ledit implant s'étendant selon un axe longitudinal (L) entre une extrémité proximale (11) apte à coopérer avec un instrument d'implantation (A) pour tenir l'implant et une extrémité distale (12) destinée à être insérée en premier dans l'os, au moins deux faces de l'implant, par exemple supérieure et inférieure, comportant chacune un plateau (13, 14) pour le contact avec les tissus osseux, chacun des plateaux comportant une portion centrale (130, 140) reliée, par l'intermédiaire d'au moins une charnière, à au moins une paire de bras de support (131, 141) orientés chacun dans des directions opposées au sein de chaque paire, un bras de chaque paire étant relié par une charnière à l'extrémité distale (11) tandis que l'autre bras est relié par une charnière à l'extrémité proximale (12), l'implant (1) étant apte à recevoir ou comportant un axe (3) central s'étendant au travers d'un manchon de coulissement à l'extrémité proximale (11) jusqu'à une bague ou une douille de traction à l'extrémité distale (12) où il est apte à transmettre une traction, lors de son actionnement par un instrument (A), sur l'extrémité distale (12) pour permettre de la rapprocher de l'extrémité proximale (11), en engendrant le pivotement des bras de support (131, 141) provoquant l'éloignement des plateaux (13, 14) l'un par rapport à l'autre et, par conséquent, l'expansion de l'implant entre la configuration repliée et la configuration déployée
**caractérisé en ce que** :
- au moins deux autres faces de l'implant, entre celles comportant les plateaux, sont couvertes d'au moins une feuille (10) par face, en alliage biocompatible de métaux, et solidarisée de manière étanche aux portions centrales (130, 140) sous les plateaux, à des faces latérales des bras (131, 141) et à des faces latérales de l'extrémité proximale (11) et de l'extrémité distale (12),
- ladite feuille (10) est déformable plastiquement pour permettre l'expansion de l'implant et présente, au moins dans la configuration repliée, une pluralité de plis antiformes (101), dit convexes, et synformes (102), dit concaves, lesdits plis étant couchés les uns sur les autres en configuration repliée, la surface totale de ladite feuille étant supérieure ou égale à la surface latérale de l'implant en configuration déployée de façon à former un compartiment étanche apte à accueillir un fluide à l'intérieur de la cavité obtenue par l'expansion de l'implant.

2. Implant selon la revendication 1, **caractérisé en ce que** la distance entre un pli synforme et le pli antiforme suivant est plus longue que la distance entre un pli antiforme et le pli synforme suivant, pour faciliter le repliement de ladite feuille sur la surface des faces latérales de l'implant en configuration repliée.

3. Implant selon l'une des revendications précédentes, **caractérisé en ce que** ladite feuille de chaque face latérale de l'implant possède, en position déployée, une forme sensiblement de losange, avec une persistance permanente d'au moins une partie des plis repliés à proximité des extrémités proximale (11) et distale (12).

4. Implant selon l'une des revendications précédentes, **caractérisé en ce que** ladite feuille est déformable plastiquement également de la configuration repliée à la configuration déployée, notamment grâce à la persistance des plis couchés aux extrémités proximale et distale, facilitant la réversibilité de l'expansion.

5. Implant selon l'une des revendications précédentes, **caractérisé en ce que** ledit axe central (3) est apte à coopérer avec et/ou se prolonge au-delà de l'extrémité distale d'un instrument (A) d'implantation au niveau de l'extrémité proximale de l'implant et possédant un conduit intérieur en communication avec un conduit (31) ménagé à l'intérieur dudit axe (3) central et débouchant dans l'espace ménagé par l'écartement des plateaux, par au moins une ouverture (32) permettant l'injection dudit fluide dans l'implant (1).

6. Implant selon l'une des revendications précédentes, **caractérisé en ce que** ladite feuille (10) est solidarisée à une face latérale de l'extrémité proximale (11) par une soudure (110) figeant l'extrémité proximale des plis couchés et repliés contre la paroi extérieure dudit manchon et/ou solidarisée à une face de l'extrémité distale (12) par une soudure (120) figeant l'extrémité distale des plis couchés et repliés contre la paroi extérieure de ladite douille.

7. Implant selon l'une des revendications précédentes, **caractérisé en ce que** les plis sont, au moins dans la configuration repliée, parallèles à l'axe longitudinal (L).

8. Implant selon l'une des revendications précédentes, **caractérisé en ce que** le nombre de plis est compris entre 4 et 16, généralement 6 à 12, de préférence de l'ordre de 8.

9. Implant selon l'une des revendications précédentes, **caractérisé en ce que** la feuille (10) possède une épaisseur comprise entre 3 et 100 microns, généralement entre 6 et 50 et de préférence 10 et 30 microns.

10. Implant selon l'une des revendications précédentes, **caractérisé en ce que** la feuille (10) est en alliage de titane.

11. Implant selon l'une des revendications précédentes, **caractérisé en ce que** la distance entre les plis est variable d'une face latérale à l'autre de l'implant, de sorte que la forme de l'implant en configuration déployée soit asymétrique transversalement à l'axe longitudinal (L).

12. Système de traitement orthopédique de tissu osseux lésé comprenant un ciment de substitution osseuse et au moins un instrument (A) d'implantation et d'injection de ciment dans l'implant, **caractérisé en ce qu'**il comporte un implant selon l'une des revendications précédentes.

13. Système selon la revendication 18, **caractérisé en ce que** l'instrument d'implantation et d'injection de ciment comporte des moyens de contrôle de la pression et/ou de l'aspiration du ciment pour replier l'implant en configuration repliée si nécessaire.

14. Système selon l'une des revendications 18 et 19, **caractérisé en ce que** l'instrument d'implantation est différent mais complémentaire de l'instrument d'injection dont le canal d'injection du ciment traverse un canal à l'intérieur d'une tige creuse de l'instrument d'implantation configurée pour tenir l'extrémité proximale de l'implant à l'extrémité distale de ladite tige creuse.

15. Procédé de fabrication d'un implant selon l'une des revendications précédentes, **caractérisé en ce qu'**il comporte :
- Obtention d'un implant expansible à deux plateaux s'écartant sous l'effet du rapprochement des extrémités de l'implant grâce à des bras supports reliant ces extrémités aux plateaux ;
- Insertion d'une feuille de matériau biocompatible métallique entre deux crémaillères munies des rainures de profil triangulaire pour imprimer des plis à la feuille ;
- Repliement des plis les uns sur les autres, pour obtenir une feuille pliée ;
- Disposition de ladite feuille pliée sur une face latérale de l'implant ;
- Solidarisation de ladite feuille sur ladite face latérale ;
- Réitération des étapes b) à e) pour l'autre face de l'implant.
